# EUROPEAN PATENT APPLICATION

(11) **EP 1 380 267 A1**
(43) Date of publication of application: **14.01.2004**
(21) Application number: 02380155.8
(22) Date of filing: 10.07.2002
(51) Int. Cl.: A61C 8/00, A61B 17/66

(54) **Surgical instrumental system for atraumatic bone distraction to affixe dental prosthesis**

(71) Applicant: Implant Microdent Systems, S.L., 08186 Llica D'Amunt (Barcelona) (ES)
(72) Inventor: Marcela,Ridao Dalmau Implant Microdent System,S.L., 08186 Llica d'Amunt, Barcelona (ES)
(74) Representative: Civanto Villar, Alicia

(57) **Abstract**

Surgical instrumental system for non-traumatic expansion of the bone to affixe dental prosthesis, with a non-traumatic effect, able to correct and wide narrow cavities, which can also be combined with lyophilized bone-providing techniques, widening the application field of implantation and atrophic area extension by means of using cylindrical parts as expanding elements, which are finished off at both ends in a cylindrical cone (2) provided with progressive and retentive conical thread with different thicknesses and lengths. This cone (2) ends up in a crest-like penetration guide point (1), which is provided with affixing means of the previous dental prosthesis, as well as with interior expansion means mechanically operated with special keys (4,5) and including signaling elements (6) and colored identification of the different thickness of the expanders indicating the depth of penetration into the gum.

## Description

This invention, a surgical instrumental system for non-traumatic expansion of the bone to affixe dental prosthesis, consists in a novel structure of a set of dental prosthesis affixing elements by means of surgical techniques, consisting in the implantation of expanders in the jawbone. These expanders consist of bases inserted into holes made in the bone and secured by pressure after activating an expansion mechanism.

The present invention will be of special interest to the manufacturing and surgical equipment supplier sector specialized in dental prosthesis.

At present, the dental applications have developed as a result of the introduction of new technologies and new sophisticated materials that have emerged, making improvements daily in the systematics of the work in placing dental implantations in order to achieve a better quality of results.

The dental implantations were conceived to solve the problems associated to the degeneration process caused by age in the teeth. Normal functioning of the teeth stimulate the surrounding bone and its tends to stand firm. However, on loosing any tooth, the bone starts to become atrophied and dissapears little by little.

Thus, one of the characteristics of old age is the loss of bone on the lower third of the face, as many people have lost their teeth and molars with the old age.

To allow this maxilare bone or jawbone to become lost is a serious inconvenience for any kind of oral rehabilitation and the only way to maintaining it is to give back its function by means of placing implantations.

Replacement of lost teeth and molar with the mentioned implantations allows permanent and comfortable recovery of all their functions (chewing, phonation, aesthetics, stabilization of the adjacent and antagonist molars, preservation of the bone in the area, etc.) and all this without having to overload the adjacent teeth, as would a removable apparatus or traditional bridge.

0n replacing a tooth with an implantation it is not only restoring the external part but also the root. Therefore, the more bone there is the better prognosis the case will have; consequently, it is not convenient to let time go by without placing the implantations.

Furthermore, implantations can replace from one tooth only up to' a number of parts in the same mouth. Other uncomfortable solutions, such as a removable prosthesis, hooks and artificial plates are eliminated with implantations.

Implantation is, therefore, one of the areas in dental science that has progressed more in the last years. More and more modern materials and techniques permit more comfortable and reliable treatment. The placing of implantations must always be carried out in the sterile environement of a theater, with highly precise motors and drills.

With tradicional techniques conical needles using by putting pressure on and hammering the bone crest to achieve expansion were utilized, with big traumatism and pain for the patient and the consequent danger of fracturing the bony structure of the jawbone, which would bear serious recovery problems.

In order to maximize the success percentage, one of the most important characteristics of all treatment of this kind is that it must be extremely non-traumatic as regards the bone and the materials used of the highest quality.

This invention has been created in order to resolve the problem of perfoming an implantation the least traumatic possible for the bone. It consist in a surgical instrumental system for non-traumatic expansion of the bone to affixe dental prosthesis, which permit simplifying the surgery during the process of placing an implantation, avoiding possible damage caused by the use of other conventional techniques.

This surgical instrumental system which is described below, consists in a set of affixing elements of the referred-to dental prosthesis by means of a controlled bone expansion system, with a non-traumatic effect, capable of correcting and widening the narrow cavities. It can also be combined with lyophilized bone-providing techniques, widening the application field of implantation and atrophic area extension. During such process the use as expansore elements of cylindrical part is made. These are finished off at both ends in a cylindrical cone provided with a conical retentive thread on the active part of the expander, with a progressive and retentive cone of different thickness and length, ending up in a crest-like penetration guide point. This enables the conical wedge to be retained when the bone is expanded, under medical control, which is a highly relevant novelty. Likewise, the latter is provided with different thickness and lenghts, and finishes in a crest-like penetration guide point, being provided with affixing means of dental prosthesis cited, as well as with interior expansion means mechanically operated by special keys and includes signaling elements and colored identification of the different thickness of the expanders indicating the depth of penetration into the gum.

Moreover, the referred-to expander of this surgical instrumental system, adopts a certain variety of lenghts depending on the zone where the implantation is placed. On the other hand, the upper part of the referred-to expanders has a polygonal housing for the different special torque keys operating the expansion mechanism.

Furthermore, for non-traumatic expansion of the bone to affixe dental prosthesis, the mentioned expanders have fast identification means consisting in color marks in the area of the point, according to a color code used for implantations.

On the other hand, this surgical instrumental system includes a number of bone compressors, with different diameters, provided with a threaded cylindrical surface with different diameters, conical point to facilitate alveolar penetration, cavity for storing and compressing the bone by raising the sinus, depth signaling to determine the length of the alveolus and therefore, the dental implantation, and polygonal storage for the torque keys.

Lastly, the described surgical instrumental system also includes other auxiliary instruments, such as fissure drills to furrow the bony crest, expansion initiating drill, manual key with lock and manual expansion key of buccal cavity in front positions.

The following is a detailed description of the proposed surgical instrumental system for non-traumatic expansion of the bone to affixe dental prosthesis, making reference to the enclosed drawings in which a preferred perfomance example of all the detail variations that do not entail a fundamental alteration of the basic characteristics of said improvements is represented for information and non-limiting purposes.

In such drawings is represented:

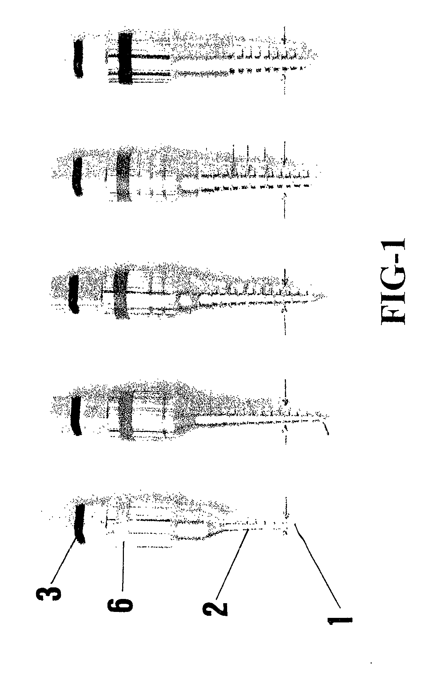

Figure 1: an elevated view of the different expanders of this surgical instrumental system for non-traumatic expansion of the bone to affixe dental prosthesis.

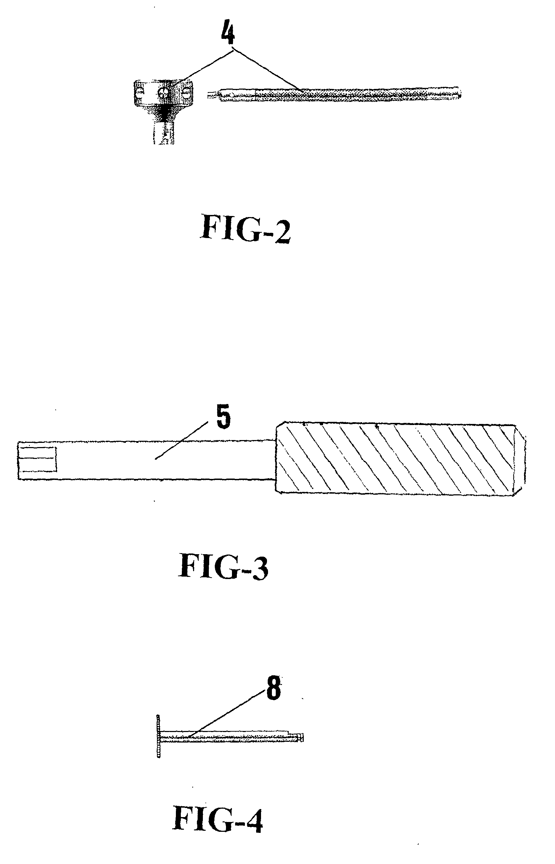

Figure 2: a side view of a manual expansion control short manual key, together with its corresponding lock, of this surgical instrumental system.
Figure 3: a side view of a long manual key for expanders, usable in front zones, in the surgical instrumental system.
Figure 4: a side view of a circular drill for longitudinal widening of the crest-like zone to be expanded in the surgical instrumental system.

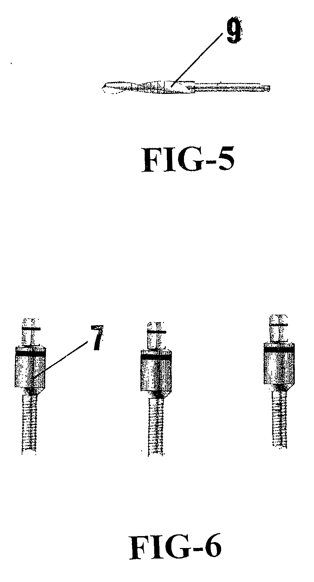

Figure 5: a side view of an initiating drill to prepare the bed of the expander in the surgical instrumental system.
Figure 6: an elevated view of various bone compressors of the surgical instrumental system for non-traumatic expansion of the bone to affixe dental prosthesis, provided with a threaded cylindrical surface with different diameters, conical point to facilitate alveolar penetration, cavity to store and compress the bone, sinus elevation, depth signaling to determine the length of the alveolus and, therefore, of the dental implantation and polygonal housing for the torque keys.

This surgical instrumental system for non-traumatic expansion of the bone to affixe dental prosthesis, the preferred perfomance example of which is described below is constitutued by a set of affixing elements of the referred-to dental prosthesis by means of a controlled, non-traumatic bone expansion procedure, capable of correcting and widening the narrow cavities. It can also be combined with lyophilized bone-providing techniques, widening the application field of implantation and atrophic area extensions. During such process the use as expander elements of cylindrical parts is made. These are finished off at both ends in a cylindrical cone (2) provided with a conical retentive thread on the active part of the expander, with a progressive and retentive cone of different thickness and lengths, ending up in a crest-like penetration guide point (1). This enables the conical wedge to be retained when the bone is expanded, under medical control, which is a highly relevant novelty. Likewise, the latter is provided with fastening means of the dental prosthesis, as well as with interior expansion means mechanically operated by special keys (4, 5) and includes signaling elements (6) and colored identification of the different thickness of the expanders indicating the depth of penetration into the gum.

Likewise, the referred-to expander (1) of this surgical instrumental system for non-traumatic expansion of the bone to affixe dental prosthesis, adopts a certain variety of lenghts based on the zone where the implantation is placed. On the other hand, the upper part of the referred-to expanders (1) has a polygonal housing for the different special torque keys (4, 5) operating the expansion mechanism.

Furthermore, for non-traumatic expansion of the bone to affixe dental prosthesis, the mentioned expanders have fast identification means consisting in color marks in the area of the point, according to a color code use for implantations.

0n the other hand, this surgical instrumental system for non-traumatic expansion of the bone to affixe dental prosthesis, includes a number of bone compressors (7), with different diameters, provided with a threaded cylindrical surface with different diameters, conical point to facilitate alveolar penetration, cavity for storing and compressing the bone by raising the sinus, depth signaling to determine the length of the alveolus, and therefore the dental implantation, and polygonal storage for the torque keys.

Lastly, the described surgical instrumental system for non-traumatic expansion of the bone to affixe dental prosthesis also includes other auxiliary instruments, such as fissure drills (8) to furrow the bony creast, expansion initiating drill (9), manual key with lock and manual expansion key of the buccal cavity in front positions.
Finally, the shape, materials and dimensions may be variable and, in general, all accesory and secondary, provided these do not alter or modify the essentiality of the improvements as described above.

## Claims

1. Surgical instrumental system for non-traumatic expansion of the bone to affixe dental prosthesis, constituted by a controlled, non-traumatic bone expansion procedure, capable of correcting and widening the narrow cavities, which can also be combined with lyophilized bone-providing techniques, widening the application field of implantation and atrophic area extension, **characterized by** the use, as expanding elements, of cylindrical pieces finished off at both ends in a cylindrical cone (2) provided with a conical retentive thread on the active part of the expander, with different thickness and lengths, ending up in a crest-like penetration guide point (1), the latter is provided with affixing means of the dental prosthesis, as well as with interior expansions means mechanically operated by special keys (4, 5) and including signaling elements (6) and colored identification of the different thickness of the expanders indicating the depth of penetration into the gum.

2. Surgical instrumental system for non-traumatic expansion of the bone to affixe dental prosthesis, according to claim 1, **characterized by** the referred-to expander (1) adopts a certain variety of lengths depending on the area where the implantation is situated.

3. Surgical instrumental system for non-traumatic expansion of the bone to affixe dental prosthesis, according to any of the precedent claims, **characterized by** the upper part of the referred-to expanders (1) includes a polygonal housing for the different special torque keys (4, 5) operating the expansion mechanism.

4. Surgical instrumental system for non-traumatic expansion of the bone to affixe dental prosthesis, according to any of the precedent claims, **characterized by** the referred-to expanders have fast identification means consisting in color marks on the area of the point, according to a color code used in implantations.

5. Surgical instrumental system for non-traumatic expansion of the bone to affixe dental prosthesis, according to any of the precedent claims, **characterized by** the described system includes a number of bone compressors (7), with different diameters, constituted by a cylindrical compressing base with one of its ends ending up in a head, provided with a mulled surface, said compressors are provided with a threaded cylindrical surface with different diameters, conical point to facilitate alveolar penetration, cavity for storing and compressing the bone, raising of the sinus, depth signaling to determine the length of the alveolus and, therefore, of the dental implantation and polygonal housing for the torque keys.

6. Surgical instrumental system for non-traumatic expansion of the bone to affixe dental prosthesis, according to any of the precedent claims, **characterized by** the described system also includes other auxiliary instruments, such as fissure drills (8), to furrow the bony crest, expansion initiating drill (9), manual key with lock and manual expansion key of the buccal cavity in front positions.
